# NEW EUROPEAN PATENT SPECIFICATION

(11) **EP 0 551 700 B2**
(45) Date of publication and mention of the opposition decision: **18.04.2007**
(45) Mention of the grant of the patent: 23.04.1997
(21) Application number: 92300406.3
(22) Date of filing: 17.01.1992
(51) Int. Cl.: A61K 9/28, A61K 9/36, A23P 1/08

(54) **Film coatings and film coating compositions based on cellulosic polymers and lactose**
Filmbeschichtungen und Beschichtzusammensetzungen auf Basis von Zellulosepolymeren und Laktose
Films de revêtement et compositions de revêtement à base de polymères cellulosiques et de lactose

(43) Date of publication of application: 21.07.1993
(73) Proprietor: BPSI Holdings, Inc., Wilmington, Delaware 19899-8985 (US); BERWIND PHARMACEUTICAL SERVICES, INC., West Point, PA 19486 (US)
(72) Inventor: Jordan, Martin Philip, Orpington, KENT BR5 2DQ (GB)
(74) Representative: Marlow, Nicholas Simon

(56) References cited:
- US-A- 4 652 313
- DATABASE WPI, accession no. 76-93359X [50], Derwent Publications Ltd, London, GB;& JP-A-51 123 815 (SHIN-ETSU CHEM. IND) 29-10-1976

## Description

### BACKGROUND OF THE INVENTION

### 1. Field of the Invention

The present invention relates to the field of aqueous film coating of pharmaceutical, food, confectionery, forms, and agricultural products, and is specifically concerned with providing coatings from a combination of a cellulosic polymer and lactose for coating such things as pharmaceutical tablets, pieces of candy, cereals, and agricultural seeds.

### 2. Description of the Prior Art

Film coating is a process of depositing a thin layer of material onto a substrate. Two goals of film coating substrates such as pharmaceutical tablets and the like are (1) to provide a functional protective barrier covering the outer surface of the substrate, and (2) to provide a pleasing appearance.

The process of film coating pharmaceutical, food, confectionery, and agricultural pieces usually involves rolling the pieces in a pan, or suspending the pieces on a cushion of air, and continuously spraying a fine mist of atomized droplets of a coating suspension onto the pieces, the droplets coalescing on the surface of the pieces to form a film coating.

Coating suspensions having an organic solvent are undesirable since these solvents are often flammable, often toxic, and hazardous to the health of film coating workings. Further, reclaiming organic solvent fumes, which are given off during spraying, from exhaust air ducting systems is expensive and often required by law.

Water-based coating suspensions are desirable to avoid the drawbacks of organic solvent-based coating suspensions. However, a major problem with aqueous coating suspensions is poor adhesion of the film to the substrate.

JP 51-123815 discloses an aqueous coating composition containing water soluble cellulose ether and saccharide, with other non-aqueous components such as plasticisers or pigments, at 1-200 parts by weight saccharide per 100 parts by weight of the cellulose ether. Lactose is given as a suitable saccharide.

JP 49-133515 discloses an aqueous coating composition containing HPMC and a saccharide at a ratio by weight of 4:1 to 1:1.

### SUMMARY OF THE INVENTION

A dry film coating composition for use in pharmaceuticals, food confectionery forms, agricultural seeds, and the like comprises a dry mixture of a cellulosic polymer and lactose, wherein the cellulosic polymer is hydroxypropyl methylcellulose (HPMC) or hydroxypropyl cellulose (HPC), in an amount from 11% to 30% by weight of the composition, preferably from 20% to 30% by weight. Optionally, the composition may include a plasticizer and/or a pigment.

A method of coating substrates such as pharmaceutical tablets, food and confectionery forms, agricultural seeds, and the like, comprises mixing a cellulosic polymer and lactose into water to form an aqueous coating suspension, wherein the cellulosic polymer is hydroxypropyl methylcellulose (HPMC) or hydroxypropyl cellulose (HPC), in an amount from 11% to 30% by weight of the non-water ingredients, preferably from 20% to 30% by weight, spraying the coating suspension onto said substrates to form a film coating on said substrates, and drying the film coating on said substrates.

The invention also includes the coated substrates, such as coated pharmaceutical tablets, and methods of making the dry film coating composition and of making a coating suspension.

Exemplary of the plasticizer are polyethylene glycol having a molecular weight of 200 to 20,000, propylene glycol, or glycerol.

Any of the pigments heretofore used in making coating dispersions for coating tablets, food, confectionery forms, agricultural seeds, and the like may be used. Examples are FD&C and D&C lakes, titanium dioxide, iron oxides or natural pigments.

The quantity of the cellulosic polymer is within a range of 11 % to 30% by weight of the dry film coating composition and of the non-water ingredients of the aqueous coating suspension. A range of 20% to 30% by weight of the dry film coating composition and of the non-water ingredients of the aqueous coating suspension is preferred.

The quantity of the lactose is within a range of about 11 % to about 56% by weight of the dry film coating composition and of the non-water ingredients of the aqueous coating suspension. A range of about 25% to 45% by weight of the dry film coating composition and of the non-water ingredients of the aqueous coating suspension is preferred.

The quantity of the plasticizer is within a range of 0% to about 30% by weight of the dry film coating composition and of the non-water ingredients of the aqueous coating suspension. A range of about 5% to 20% by weight of the dry film coating composition and of the non-water ingredients of the aqueous coating suspension is preferred.

The quantity of the pigment is within a range of 0% to about 55% by weight of the dry film coating composition and of the non-water ingredients of the aqueous coating suspension. A range of about 15% to 40% by weight of the dry film coating composition and of the non-water ingredients of the aqueous coating suspension is preferred.

A preferred formula for the present inventive dry film coating composition is:

| **COMPONENT** | **% w/w** |
|---|---|
| CELLULOSIC POLYMER | 26.80 |
| LACTOSE | 40.20 |
| PLASTICIZER | 8.00 |
| PIGMENT | 25.00 |

The following examples of the invention all disclose formulations which may be mixed into water to form an aqueous coating suspension effective to coat pharmaceutical tablets, food and confectionery pieces, and agricultural seeds. Seeds are advantageously coated to meet various needs, such as color coating for identification purposes, adhesion of various additives, (e.g., pest control agents and inocula), prevention of handling damage, and facilitating the use of mechanical planting equipment The coated forms include medicinal tablets, vitamin tablets, aspirin tablets, capsules, chewing gum balls, candy pieces, breakfast cereals, and agricultural seeds.

### EXAMPLES

The following examples illustrate the invention. All units and percentages used herein are by weight.

### EXAMPLE 1

67.00 grams of hydroxypropyl methylcellulose (Methocel E15 made by Dow Chemical Company), 100.50 grams of lactose, 60.00 grams of titanium dioxide, and 2.50 grams of Indigo carmine aluminum lake 14% are loaded into a dry powder blender such as a P.K. blender and mixed Vigorously for 25 minutes to form an homogenous mixture. Then, 20.00 grams of a plasticizer, polyethylene glycol 4000, (PEG 4000 made by Union Carbide), is added to the homogenous mixture and gently blended into it

Optionally, the homogenous mixture containing the plasticizer is granulated using a planetary mixer, such as a Hobart planetary mixer. After the dry film coating composition is loaded into the mixer and the mixer is switched on, sufficient water is slowly added until the composition forms slightly adherent granules. These granules are then passed through a 1-2 mm screen and then dried in a 30°C oven until the moisture content is below 5%. The composition is then sieved again through a 1-2mm screen and is then ready for use in a non-dusting, granular form. If not optionally granulated in a planetary mixer, the powder may be milled such as in a hammer mill (Apex Machinery, Dartford, England), for example.

Other methods of granulation which may be used are spray granulation and roller compaction.

The following examples 2 to 17, which show different formulations of the dry film coating composition of the invention, further illustrate the invention. In each example 2 to 17; a dry film coating composition is made using the procedure of Example 1. Methocel E15 and Methocel E50 are hydroxypropyl methylcellulose (HPMC) made by Dow Chemical Company. PEG 200, PEG 400, PEG 4000, PEG 8000, and PEG 20000 are polyethylene glycol made by Union Carbide. Klucel EF is hydroxypropyl cellulose made by Hercules Co.

### EXAMPLE 2

| **COMPONENT** | **% w/w** | **grams** |
|---|---|---|
| HPMC (Methocel E15) | 20.39 | 50.98 |
| LACTOSE | 30.58 | 76.44 |
| PEG 4000 | 30.00 | 75.00 |
| Ti02 | 18.27 | 45.68 |
| INDIGO CARMINE ALUMINUM LAKE 14% | 0.76 | 1.90 |
| | 100.00 | 250.00 |

### EXAMPLE 3

| **COMPONENT** | **% w/w** | **grams** |
|---|---|---|
| HPMC (Methocel E15) | 16.00 | 40.00 |
| LACTOSE | 24.00 | 60.00 |
| PEG 4000 | 14.55 | 36.38 |
| Ti02 | 43.64 | 109.10 |
| INDIGO CARMINE ALUMINUM LAKE 14% | 1.81 | 4.52 |
| | 100.00 | 250.00 |

### EXAMPLE 4

| **COMPONENT** | **% w/w** | **grams** |
|---|---|---|
| HPMC (Methocel E15) | 11.17 | 27.93 |
| LACTOSE | 55.83 | 139.57 |
| PEG 4000 | 8.00 | 20.00 |
| Ti02 | 24.00 | 60.00 |
| INDIGO CARMINE ALUMINUM LAKE 14% | 1.00 | 2.50 |
| | 100.00 | 250.00 |

### EXAMPLE 5

| **COMPONENT** | **% w/w** | **grams** |
|---|---|---|
| HPMC (Methocel E15) | 15.66 | 39.15 |
| LACTOSE | 24.00 | 60.00 |
| PEG 4000 | 5.34 | 13.35 |
| TiO2 | 52.80 | 132.00 |
| INDIGO CARMINE ALUMINUM LAKE 14% | 2.20 | 5.50 |
| | 100.00 | 250.00 |

### EXAMPLE 6

| **COMPONENT** | **% w/w** | **grams** |
|---|---|---|
| HPMC (Methocel E15) | 26.80 | 67.00 |
| LACTOSE | 40.20 | 100.50 |
| PEG 4000 | 8.00 | 20.00 |
| IRON OXIDE YELLOW | 25.00 | 62.50 |
| | 100.00 | 250.00 |

### EXAMPLE 7

| **COMPONENT** | **% w/w** | **grams** |
|---|---|---|
| HPMC (Methocel E15) | 26.80 | 67.00 |
| LACTOSE | 40.20 | 100.50 |
| PEG 4000. | 8.00 | 20.00 |
| TiO2 | 24.00 | 60.00 |
| IRON OXIDE YELLOW | 1.00 | 2.50 |
| | 100.00 | 250.00 |

### EXAMPLE 8

| **COMPONENT** | **% w/w** | **grams** |
|---|---|---|
| HPMC (Methocel E15) | 26.80 | 67.00 |
| LACTOSE | 40.20 | 100.50 |
| PEG 4000 | 8.00 | 20.00 |
| TiO2 | 1.00 | 2.50 |
| INDIGO CARMINE ALUMINUM LAKE 14% | 24.00 | 60.00 |
| | 100.00 | 250.00 |

### EXAMPLE 9

| **COMPONENT** | **% w/w** | **grams** |
|---|---|---|
| HPMC(Methocel E15) | 26.80 | 67.00 |
| LACTOSE | 40.20 | 100.50 |
| PEG 400 | 8.00 | 20.00 |
| TiO2 | 24.00 | 60.60 |
| INDIGO CARMINE ALUMINUM LAKE 14% | 1.00 | 2.50 |
| | 100.00 | 250.00 |

### EXAMPLES 10

| **COMPONENT** | **% w/w** | **grams** |
|---|---|---|
| HPMC (Methocel E15) | 26.80 | 67.00 |
| LACTOSE | 40.20 | 100.50 |
| PROPYLENE GLYCOL | 8.00 | 20.00 |
| TiO2 | 24.00 | 60.00 |
| INDIGO CARMINE ALUMINUM LAKE 14% | 1.00 | 2.50 |
| | 100.00 | 250.00 |

### EXAMPLE 11

| **COMPONENT** | **% w/w** | **grams** |
|---|---|---|
| HPMC(Methocel E15) | 26.80 | 67.00 |
| LACTOSE | 40.20 | 100.50 |
| GLYCEROL | 8.00 | 20.00 |
| TiO2 | 24.00 | 60.00 |
| INDIGO CARMINE ALUMINUM LAKE 14% | 1.00 | 2.50 |
| | 100.00 | 250.00 |

### EXAMPLE 12

| **COMPONENT** | **% w/w** | **grams** |
|---|---|---|
| HPC (Klucel EF) | 26.80 | 67.00 |
| LACTOSE | 40.20 | 100.50 |
| PEG4000 | 8.00 | 20.00 |
| Ti02 | 24.00 | 60.00 |
| INDIGO CARMINE ALUMINUM LAKE 14% | 1.00 | 2.50 |
| | 100.00 | 250.00 |

### EXAMPLE 13

| **COMPONENT** | **% w/w** | **grams** |
|---|---|---|
| HPMC (Methocel E50) | 20.83 | 52.07 |
| LACTOSE | 41.67 | 104.17 |
| PEG 4000 | 6.25 | 15.63 |
| TiO2. | 30.00 | 75.00 |
| INDIGO CARMINE ALUMINUM LAKE 14% | 1.25 | 3.13 |
| | 100.00 | 250.00 |

### EXAMPLE 14

| **COMPONENT** | **% w/w** | **grams** |
|---|---|---|
| HPMC (Methocel E15) | 26.80 | 67.00 |
| LACTOSE | 40.20 | 100.50 |
| PEG 8000 | 8.00 | 20.00 |
| TiO2 | 24.00 | 60.00 |
| TARTRAZINE ALUMINUM LAKE 25% | 1.00 | 2.50 |
| | 100.00 | 250.00 |

### EXAMPLE 15

| **COMPONENT** | **% w/w** | **grams** |
|---|---|---|
| HPMC (Metnocel E15) | 26.80, | 67.00 |
| LACTOSE | 40.20 | 100.50 |
| PEG 20,000 | 8.00 | 20.00 |
| TiO2 | 24.00 | 60.00 |
| INDIGO CARMINE ALUMINUM LAKE 14% | 1.00 | 2.50 |
| | 100.00 | 250.00 |

### EXAMPLE 16

| **COMPONENT** | **% w/w** | **grams** |
|---|---|---|
| HPMC (Methocel E15) | 26.80 | 67.00 |
| LACTOSE | 40.20 | 100.50 |
| PEG 200 | 8.00 | 20.00 |
| TiO2 | 24.00 | 60.00 |
| INDIGO CARMINE ALUMINUM LAKE 14% | 1.00 | 2.50 |
| | 100.00 | 250.00 |

### EXAMPLE 17

A dry film coating composition having the following formula is made as in example 1, except that no plasticizer is blended into the composition:

| **COMPONENT** | **% w/w** | **grams** |
|---|---|---|
| HPMC (Methocel E15) | 29.13 | 72.82 |
| LACTOSE | 43.70 | 109.25 |
| TiO2 | 26.08 | 65.20 |
| INDIGO CARMINE ALUMINUM LAKE 14% | 1.09 | 2.73 |
| | 100.00 | 250.00 |

Each of the dry film coating compositions of Examples 1-17 is constituted in water to form a coating suspension. In each case, 170 grams of the dry film coating composition is dispersed into 830 grams of purified water. Preferably, this is accomplished by placing the 830 grams of purified water into a mixing vessel having the diameter that is about equal to the depth of the final suspension. Then, a low shear mixer is lowered into the water and turned on. Preferably, the mixing head of the mixer is about one third the diameter of the mixing vessel and creates a vortex from the edge of the vessel down to just above the mixing head. The 170 grams of the dry film coating composition is then added to the vortex at a rate where there is no excessive build-up of the dry film coating composition. The speed and depth of the mixing head is adjusted to avoid air being drawn into the suspension which would result in foaming. The suspension is stirred for 45 minutes and is then ready for spraying.

The inventive coating suspensions are then sprayed onto medicinal tablets, vitamin tablets, aspirin tablets, capsules, chewing gum balls, candy pieces, breakfast cereals, and agricultural seeds, and allowed to dry. The film coatings so produced have an excellent appearance and adhere extremely well to the substrates.

Preferably, the inventive film coating suspension is formed by blending together all the dry ingredients of the coating formula, and then dispersing the mixture of the dry ingredients into water. However, the film coating suspension may be prepared by stirring the ingredients of the coating formulation one by one into water to form a coating suspension.

Further, the inventive coating suspension may be spray-granulated to form a dry edible film coating composition that may be remixed into water when desired to form a coating suspension.

The inclusion of lactose in the coating composition dramatically and unexpectedly improves adhesion of the film coating onto substrates such as pharmaceuticals, food, confectionery forms, agricultural seeds, and the like.

The dry film coating composition of the invention when constituted in water has a viscosity lower than that of a conventional polymer coating system having the same total solids. For an equivalent final dispersion viscosity as a conventional polymer coating system, a coating dispersion may be produced under the invention which has a higher solids loading, and consequently a lower solvent content Accordingly, since the film coating suspensions of the invention may be sprayed at a higher solids level, spraying times are lower than the spraying times for conventional systems, which results in lower processing time cost.

Most cellulosic polymers show some degree of tack as they dry on the substrate surface. This tack is reduced with the inclusion of lactose in the inventive formula, thus further facilitating faster processing times, and thereby lowering costs.

The inclusion of lactose in the inventive formulation improves the light stability of organic pigments and natural colors commonly used in film coatings.

The inclusion of lactose in the formulation has the effect of reducing the moisture vapor permeability of the film.

## Claims

1. A dry film coating composition for use in pharmaceuticals, food, confectionery forms, agricultural seeds, and the like, comprising:
a cellulosic polymer and lactose, wherein the cellulosic polymer is hydroxypropyl methylcellulose (HPMC) or hydroxypropyl cellulose (HPC), in an amount from 11% to 30% by weight of the composition.

2. A dry film composition according to claim 1, comprising hydroxypropyl methylcellulose (HPMC) or hydroxypropyl cellulose (HPC) in an amount from 20% to 30% by weight of the composition.

3. A dry film composition according to claim 1 or 2, further comprising a plasticizer.

4. A dry film coating composition according to claim 3, in which the plasticizer is polyethylene glycol 200 to 20,000, propylene glycol, or glycerol.

5. A dry film coating composition according to claim 3 or 4, in which the plasticizer content is up to 30% by weight of the composition.

6. A dry film coating composition according to any preceding claim, further comprising a pigment.

7. A dry film coating composition according to claim 6 in which the pigment is at least one of FD&C lakes, D&C lakes, titanium dioxide, iron oxides, or natural pigments.

8. A dry film coating composition according to claim 6 or 7, in which the pigment content is up to 55% by weight of the composition.

9. A dry film coating composition according to any preceding claim, in which the lactose content is from 11% to 20% by weight of the composition.

10. A method of coating substrates such as pharmaceutical tablets, food and confectionery forms, agricultural seeds, comprising:
mixing hydroxypropyl methylcellulose (HPMC) or hydroxypropyl cellulose (HPC) and lactose into water
to form an aqueous coating suspension, the hydroxypropyl methylcellulose (HPMC) or hydroxypropyl cellulose (HPC) forming from 11% to 30% by weight of the non-water ingredients;
spraying the coating suspension onto the substrates to form a film coating on the substrates; and
drying the film coating on said substrates.

11. The method according to claim 10, wherein the hydroxypropyl methylcellulose (HPMC) or hydroxypropyl cellulose (HPC) forms from 20% to 30% by weight of the non-water ingredients.

12. A method of making a dry film coating composition for use in coating pharmaceutical tablets, food, confectionery forms, agricultural seeds, comprising mixing hydroxypropyl methylcellulose (HPMC) or hydroxypropyl cellulose (HPC) and lactose together to form a dry film coating composition, the hydroxypropyl methylcellulose (HPMC) or hydroxypropyl cellulose (HPC) forming from 11% to 30% by weight of the composition.

13. The method according to claim 12, wherein the hydroxypropyl methylcellulose (HPMC) or hydroxypropyl cellulose (HPC) forms from 20% to 30% by weight of the composition.

14. A method according to claim 12 or 13, further comprising granulating the dry film coating: composition.

15. A method of making a dry film coating composition for use in coating pharmaceutical tablets, food and confectionery forms, agricultural seeds, comprising mixing hydroxypropyl methylcellulose (HPMC) or hydroxypropyl cellulose (HPC) and lactose into water to form an aqueous coating suspension, the hydroxypropyl methylcellulose (HPMC) or hydroxypropyl cellulose (HFC) forming from 11% to 30% by weight of the composition, and spray granulating the aqueous coating suspension to form a dry film coating composition.

16. The method according to claim 15, wherein the hydroxypropyl methylcellulose (HPMC) or hydroxypropyl cellulose (HPC) forms from 20% to 30% by weight of the composition.

17. A method according to claims 10, 11, 15 or 16, further comprising adding a plasticizer to the aqueous coating suspension.

18. A method according to claims 12, 7.3. or 14 further comprising mixing-a plasticizer with the hydroxypropyl methylcellulose (HPMC) or hydroxypropyl cellulose (HPC) and the lactose until blended to form the dry film coating composition.

19. A method according to claims 17 or 18, in which the plasticizer is polyethylene glycol 200 to 20,000, propylene glycol, or glycerol.

20. A method according to claim 17, 18, or 19, in which the plasticizer content is up to 30% by weight of the non-water ingredients of the composition.

21. A method according to claims 10, 11, 15, 16 and 17 or to claim 19 or 20 when dependent on any of claims 10, 11, 15, 16, and 17, further comprising dispersing a pigment into the coating suspension.

22. A method according to claims 12, 13, 14, or 18 or to claim 19 or 20 when dependent on claim 12, 13, 14, or 18, further comprising adding a pigment to the mixture and mixing until the combined mix is blended to form the dry film coating composition.

23. A method according to any of claims 21 or 22 in which the pigment is at least one of the FD&C lakes, D&C lakes, titanium dioxide, iron oxides, or natural pigments.

24. A method according to claims 21, 22 or 23 in which the pigment content is up to 55% by weight of the non-water ingredients of the coating suspension.

25. A method according to.any of claims 10 to 24, in which the lactose content is from 11% to 55% by weight of the non-water ingredients of the coating suspension.

26. An aqueous coating suspension for coating substrates such as pharmaceutical tablets, food and confectionery forms, agricultural seeds, comprising a mixture of hydroxypropyl methylcellulose (HPMC) or hydroxypropyl cellulose (HPC), lactose, and water, the hydroxypropyl methylcellulose (HFMC), or hydroxypropyl cellulose (HPC) forming from 11% to 30% by weight of the non-water ingredients.

27. An aqueous coating suspension according to claim 26, in which the hydroxypropyl methylcellulose (HPMC) or hydroxypropyl cellulose (HPC) forms from 20% to 30% by weight of the non-water ingredients.

28. An aqueous coating suspension according to claim 26 or 27, further comprising a plasticizer.

29. An aqueous coating suspension according to claim 28, in which the plasticizer is polyethylene glycol 200 to 20,000, propylene glycol, or glycerol.

30. An aqueous coating suspension according to claims 28 or 29, in which the plasticizer content is up to 30% by weight of the non-water ingredients of the coating suspension.

31. An aqueous coating suspension according to any of claims 26 to 30, further comprising a pigment.

32. An aqueous coating suspension according to claim 31, in which the pigment is at least one of FD&D lakes, D&C lakes, titanium dioxide, iron oxides, or natural pigments.

33. An aqueous coating suspension according to claims 31 or 32, in which the pigment content is up to 55% by weight of the non-water ingredients of the coating suspension.

34. An aqueous coating suspension according to any of claims 26 to 33, in which the lactose content is 11 to 56% by weight of the non-water ingredients of the coating suspension.

## Patentansprüche

1. Trockene Überzugszusammensetzung zur Verwendung bei Pharmazeutika, Lebensmitteln, Konfektformen, landwirtschaftlichen Samen und dergleichen, enthaltend ein Cellulosepolymer und Lactose, wobei das Cellulosepolymer Hydroxypropylmethylcellulose (HPMC) oder Hydroxypropylcellulose (HPC) mit einem Anteil von 11 bis 30 Gewichtsprozent der Zusammensetzung ist.

2. Trockene Überzugszusammensetzung gemäß Anspruch 1, enthaltend Hydroxypropylmethylcellulose (HPMC) oder Hydroxypropylcellulose (HPC) mit einem Anteil von 20 bis 30 Gewichtsprozent der Zusaammensetzung.

3. Trockene Überzugszusammensetzung gemäß Anspruch 1 oder 2, weiter enthaltend einen Weichmacher.

4. Trockene Überzugszusammensetzung gemäß Anspruch 3, wobei der Weichmacher Polyethylenglycol 200 bis 20.000, Propylenglycol oder Glycerol ist.

5. Trockene Überzugszusammensetzung gemäß Anspruch 3 oder 4, wobei der Weichmachergehalt bis zu 30 Gewichtsprozent der Zusammensetzung ist.

6. Trockene Überzugszusammensetzung gemäß einem der vorhergehenden Ansprüche, weiter enthaltend ein Pigment.

7. Trockene Überzugszusammensetzung gemäß Anspruch 6, wobei das Pigment wenigstens eines von FD&C-Lacken, D&C-Lacken, Titandioxid, Eisenoxiden oder natürlichen Pigmenten ist .

8. Trockene Überzugszusammensetzung gemäß Anspruch 6 oder 7, wobei der Pigmentgehalt bis zu 55 Gewichtsprozent der Zusammensetzung ist.

9. Trockene Überzugszusammensetzung gemäß einem der vorhergehenden Ansprüche, wobei der Lactosegehalt 11 bis.20 Gewichtsprozent der Zusammensetzung ist.

10. Verfahren zum Überziehen von Trägern wie pharmazeutischen Tabletten, Lebensmittel- und Konfektformen, landwirtschaftlichen Samen, enthaltend:
Vermischen von Hydroxypropylmethylcellulose (HPMC) oder Hydroxypropylcellulose (HPC) und von Lactose in Wasser, um eine wässrige Überzugssuspension zu bilden, wobei Hydroxypropylmethylcellulose (HPMC) oder Hydroxypropylcellulose (HPC) 11 bis 30 Gewichtsprozent der Nicht-Wasser-Inhaltsstoffe bildet,
Aufsprühen der Überzugssuspension auf die Träger, um auf den Trägern einen Filmüberzug zu bilden, und
Trocknen des Filmüberzugs auf den Trägern.

11. Verfahren gemäß Anspruch 10, wobei Hydroxypropylmethylcellulose (HPMC) oder Hydroxypropylcellulose (HPC) 20 bis 30 Gewichtsprozent der Nicht-Wasser-Inhaltsstoffe bildet.

12. Verfahren zur Herstellung einer trockenen Überzugszusammensetzung zur Verwendung beim Überziehen von pharmazeutischen Tabletten, Lebensmittel- und Konfektformen, landwirtschaftlichen Samen, enthaltend:
Vermischen von Hydroxypropylmethylcellulose (HPMC) oder Hydroxypropylcellulose (HPC) mit Lactose, um eine trockene Überzugszusammensetzung zu bilden, wobei die Hydroxypropylmethylcellulose (HPMC) oder Hydroxypropylcellulose (HPC) 11 bis 30 Gewichtsprozent der Zusammensetzung bildet.

13. Verfahren nach Anspruch 12, wobei die Hydroxypropylmethylcellulose (HPMC) oder Hydroxypropylcellulose (HPC) 20 bis 30 Gewichtsprozent der Zusammensetzung bildet.

14. Verfahren gemäß Anspruch 12 oder 13, weiter enthaltend das Granulieren der trockenen Überzugszusammensetzung.

15. Verfahren zur Herstellung einer trockenen Überzugszusammensetzung zur Verwendung beim Überziehen pharmazeutischer Tabletten, Lebensmittel- und Konfektformen, landwirtschaftlichen Samen, enthaltend:
Vermischen von Hydroxypropylmethylcellulose (HPMC) oder Hydroxypropylcellulose (HPC) und von Lactose in Wasser, um eine wässrige Überzugssuspension zu bilden, wobei die Hydroxypropylmethylcellulose (HPMC) oder Hydroxypropylcellulose (HPC) 11 bis 30 Gewichtsprozent der Zusammensetzung bildet, und
Sprühgranulieren der wässrigen Überzugssuspension, um eine trockene Überzugszusammensetzung zu bilden.

16. Verfahren nach Anspruch 15, wobei die Hydroxypropylmethylcellulose (HPMC) oder Hydroxypropylcellulose (HPC) 20 bis 30 Gewichtsprozent der Zusammensetzung bildet.

17. Verfahren gemäß Ansprüchen 10, 11, 15 oder 16, weiter enthaltend das Hinzufügen eines Weichmachers zur wässrigen Überzugssuspension.

18. Verfahren gemäß Anspruch 12, 13 oder 14, weiter enthaltend das Vermischen eines Weichmachers mit der Hydroxypropylmethylcellulose (HPMC) oder Hydroxypropylcellulose (HPC) und der Lactose, bis sie vermischt sind, um die trockene Überzugszusammensetzung zu bilden.

19. Verfahren gemäß Anspruch 17 oder 18, weiter enthaltend, daß der Weichmacher Polyethylenglycol 200 bis 20.000, Propylenglycol oder Glycerol ist.

20. Verfahren gemäß Anspruch 17, 18 oder 19, wobei der Weichmachergehalt bis zu 30 Gewichtsprozent der Nicht-Wasser-Inhaltsstoffe der Zusammensetzung ist.

21. Verfahren gemäß einem der Ansprüche 10, 11, 15, 16 und 17 oder gemäß Anspruch 19 oder 20, soweit er von einem der Ansprüche 10, 11, 15, 16 und 17 abhängt, weiter enthaltend:
Dispergieren eines Pigments in der Überzugssuspension.

22. Verfahren gemäß Anspruch 12, 13, 14 oder 18 oder gemäß Anspruch 19 oder 20, soweit abhängig von Anspruch 12, 13, 14 oder 18, weiter enthaltend:
Zufügen eines Pigments zur Mischung und Vermischen, bis die zusammengesetzte Mischung vermischt ist, um die trockene Überzugszusammensetzung zu bilden.

23. Verfahren gemäß Anspruch 21 oder 22, wobei das Pigment wenigstens eines von FD&C-Lacken, D&C-Lacken, Titandioxid, Eisenoxiden oder natürlichen Pigmenten ist.

24. Verfahren gemäß Anspruch 21, 22 oder 23, wobei der Pigmentgehalt bis zu 55 Gewichtsprozent der Nicht-Wasser-Inhaltsstoffe der Überzugssuspension ist.

25. Verfahren gemäß einem der Ansprüche 10 bis 24, wobei der Lactosegehalt 11 bis 56 Gewichtsprozent der Nicht-Wasser-Inhaltsstoffe der Überzugssuspension ist.

26. Wässrige Überzugssuspension zum Überziehen von Trägern wie pharmazeutischen Tabletten, Lebensmittel- und Konfektformen, landwirtschaftlichen Samen, enthaltend eine Mischung aus Hydroxypropylmethylcellulose (HPMC) oder Hydroypropylcellulose (HPC), Lactose und Wasser, wobei die Hydroxypropylmethylcellulose (HPMC) oder Hydroypropylcellulose (HPC) 11 bis 30 Gewichtsprozent der Nicht-Wasser-Inhaltsstoffe bildet.

27. Wässrige Überzugssuspension gemäß Anspruch 26, in der die Hydroxypropylmethylcellulose (HPMC) oder Hydroypropylcellulose (HPC) 20 bis 30 Gewichtsprozent der Nicht-Wasser-Inhaltsstoffe bildet.

28. Wässrige Überzugssuspension gemäß Anspruch 26 oder 27, weiter enthaltend: einen Weichmacher.

29. Wässrige Überzugssuspension gemäß Anspruch 28, wobei der Weichmacher Polyethylenglycol 200 bis 20.000, Propylenglycol oder Glycerol ist.

30. Wässrige Überzugssuspension gemäß Anspruch 28 oder 29, wobei der Weichmachergehalt bis zu 30 Gewichtsprozent der Nicht-Wasser-Inhaltsstoffe der Überzugssuspension ist.

31. Wässrige Überzugssuspension gemäß einem der Ansprüche 26 bis 30, weiter enthaltend: ein Pigment.

32. Wässrige Überzugssuspension gemäß Anspruch 31, wobei das Pigment wenigstens eines von FD&C-Lacken, D&C-Lacken, Titandioxid, Eisenoxiden oder natürlichen Pigmenten ist.

33. Wässrige Überzugssuspension gemäß Anspruch 31 oder 32, wobei der Pigmentgehalt bis zu 55 Gewichtsprozent der Nicht-Wasser-Inhaltsstoffe der Überzugssuspension ist.

34. Wässrige Überzugssuspension gemäß einem der Ansprüche 26 bis 33, wobei der Lactosegehalt 11 bis 56 Gewichtsprozent der Nicht-Wasser-Inhaltsstoffe der Überzugssuspension ist.

## Revendications

1. Composition sèche de film de revêtement pour une utilisation dans les produits pharmaceutiques, les produits d'alimentation, les formes de confiserie, les graines agricoles, et analogues, comprenant un polymère cellulosique et du lactose, dans lequel le polymère cellulosique est l'hydroxypropylméthylcellulose (HPMC) ou l'hydroxypropyl cellulose (HPC), constituant de 11% à 30% en poids de la composition.

2. Composition sèche de film de revêtement selon la revendication 1, comprenant de l'hydroxypropylméthylcellulose (HPMC) ou de l'hydroxypropyl cellulose (HPC) à raison de 20% à 30% en poids de la composition.

3. Composition sèche de film de revêtement selon la revendication 1 ou 2, comprenant de plus un plastifiant.

4. Composition sèche de film de revêtement selon la revendication 3, dans laquelle le plastifiant est un polyéthylèneglycol 200 à 20 000, le propylèneglycol ou le glycérol.

5. Composition sèche de film de revêtement selon la revendication 3 ou 4, dans laquelle la teneur en plastifiant constitue jusqu'à 30% en poids de la composition.

6. Composition sèche de film de revêtement selon l'une quelconque des revendications précédentes, comprenant de plus un pigment.

7. Composition sèche de film de revêtement selon la revendication 6, dans laquelle le pigment est au moins un pigment choisi parmi les laques FD&C, les laques D&C, le dioxyde de titane, les oxydes de fer ou les pigments naturels.

8. Composition sèche de film de revêtement selon la revendication 6 ou 7, dans laquelle la teneur en pigment constitue jusqu'à 55% en poids de la composition.

9. Composition sèche de film de revêtement selon l'une quelconque des revendications précédentes, dans laquelle la teneur en lactose est de 11 % à 20% en poids de la composition.

10. Procédé pour revêtir des substrats tels que les comprimés pharmaceutiques, les formes d'alimentation et de confiserie, les graines agricoles, consistant:
- à mélanger de l'hydroxypropylméthylcellulose (HPMC) ou de l'hydroxypropyl cellulose (HPC) et du lactose dans de l'eau pour former une suspension aqueuse de revêtement, l'hydroxypropylméthylcellulose (HPMC) ou l'hydroxypropyl cellulose (HPC) formant de 11 % à 30% en poids des ingrédients non aqueux;
- à pulvériser la suspension de revêtement sur les substrats pour former un revêtement en film sur les substrats; et
- à sécher le revêtement en film sur lesdits substrats.

11. Procédé selon la revendication 10, dans lequel l'hydroxypropylméthylcellulose (HPMC) ou l'hydroxypropyl cellulose (HPC) forme de 20% à 30% en poids des ingrédients non aqueux.

12. Procédé de fabrication d'une composition sèche de film de revêtement pour une utilisation dans le film de revêtement de comprimés pharmaceutiques, de formes d'alimentation et de confiserie, de graines agricoles, consistant :
- à mélanger ensemble de l'hydroxypropylméthylcellulose (HPMC) ou de l'hydroxypropyl cellulose (HPC)et du lactose pour former une composition sèche de film de revêtement, l'hydroxypropylméthylcellulose (HPMC) ou l'hydroxypropyl cellulose (HPC)formant de 11% à 30% en poids de la composition.

13. Procédé selon la revendication 12, dans lequel l'hydroxypropylméthylcellulose (HPMC) ou l'hydroxypropyl cellulose (HPC) forme de 20% à 30% en poids de la composition.

14. Procédé selon la revendication 12 ou 13, consistant de plus à granuler la composition sèche de film de revêtement.

15. Procédé de fabrication d'une composition sèche de film de revêtement pour une utilisation dans le revêtement de comprimés pharmaceutiques, de formes d'alimentation et de confiserie, de graines agricoles, consistant:
- à mélanger de l'hydroxypropylméthylcellulose (HPMC) ou de l'hydroxypropyl cellulose (HPC)et du lactose dans de l'eau pour former une suspension aqueuse de revêtement, l'hydroxypropylméthylcellulose (HPMC) ou l'hydroxypropyl cellulose (HPC)formant de 11% à 30% en poids de la composition et
- à granuler par pulvérisation la suspension aqueuse de revêtement pour former une composition sèche de film de revêtement.

16. Procédé selon la revendication 15, dans lequel l'hydroxypropylméthylcellulose (HPMC) ou l'hydroxypropyl cellulose (HPC) forme de 20% à 30% en poids de la composition.

17. Procédé selon la revendication 10, 11, 15 ou 16, consistant de plus à ajouter un plastifiant à la suspension aqueuse de revêtement.

18. Procédé selon la revendication 12, 13 ou 14, consistant de plus à mélanger un plastifiant avec l'hydroxypropylméthylcellulose (HPMC) ou l'hydroxypropyl cellulose (HPC) et le lactose jusqu'à homogénéisation pour former la composition sèche de revêtement en film,

19. Procédé selon la revendication 17 ou 18 dans lequel le plastifiant ajouté est un polyéthylèneglycol 200 à 20 000, le propylèneglycol ou le glycérol.

20. Procédé selon la revendication 17, 18 ou 19, dans lequel la teneur en plastifiant constitue jusqu'à 30% en poids des ingrédients non aqueux de la composition.

21. Procédé selon l'une quelconque des revendications 10, 11, 15, 16 et 17 ou selon la revendication 19 ou 20 quand elles dépendent de l'une quelconque des revendications 10, 11, 15, 16 et 17, consistant de plus à disperser un pigment dans la suspension de revêtement.

22. Procédé selon la revendication 12, 13, 14 ou 18 ou selon la revendication 19 ou 20 quand elles dépendent de la revendication 12, 13, 14 ou 18, consistant de plus à ajouter un pigment au mélange et à mélanger jusqu'à ce que le mélange combiné soit homogène pour former la composition sèche de film de revêtement.

23. Procédé selon la revendication 21 ou 22, dans lequel le pigment est au moins un pigment choisi parmi les laques FD&C; les laques D&C, le dioxyde de titane, les oxydes de fer ou les pigments naturels.

24. Procédé selon la revendication 21, 22 ou 23, dans lequel la teneur en pigment constitue jusqu'à 55% en poids des ingrédients non aqueux de la suspension de revêtement.

25. Procédé selon l'une quelconque des revendications 10 à 24, dans lequel la teneur en lactose est de 11% à 56% en poids des ingrédients non aqueux de la suspension de revêtement.

26. Suspension aqueuse de revêtement pour revêtir des substrats tels que les comprimés pharmaceutiques, les formes d'alimentation et de confiserie, les graines agricoles, comprenant un mélange:
- de l'hydroxypropylméthylcellulose (HPMC) ou de l'hydroxypropyl cellulose (HPC),
- de lactose, et
- d'eau,
l'hydroxypropylméthylcellulose (HPMC) ou l'hydroxypropyl cellulose (HPC) formant 11% à 30% en poids des ingrédients non aqueux.

27. Suspension aqueuse de revêtement selon la revendication 26, dans laquelle l'hydroxypropylméthylcellulose (HPMC) ou l'hydroxypropyl cellulose (HPC)forme de 20% à 30% en poids des ingrédients non aqueux.

28. Suspension aqueuse de revêtement selon la revendication 26 ou 27, comprenant de plus un plastifiant.

29. Suspension de revêtement aqueuse selon la revendication 28, dans laquelle le plastifiant est un polyéthylèneglycol 200 à 20 000, le propylèneglycol ou le glycérol.

30. Suspension aqueuse de revêtement selon la revendication 28 ou 29, dans laquelle la teneur en plastifiant constitue jusqu'à 30% en poids des ingrédients non aqueux de la suspension de revêtement.

31. Suspension aqueuse de revêtement selon l'une quelconque des revendications 26 à 30, comprenant de plus un pigment.

32. Suspension de revêtement aqueuse selon la revendication 31, dans laquelle le pigment est au moins un pigment choisi parmi les laques FD&C, les laques D&c, le dioxyde de titane, les oxydes de fer ou les pigments naturels.

33. Suspension aqueuse de revêtement selon la revendication 31 ou 32, dans laquelle la teneur en pigment constitue jusqu'à 55% en poids des ingrédients non aqueux de la suspension de revêtement.

34. Suspension aqueuse de revêtement selon l'une quelconque des revendications 26 à 33, dans laquelle la teneur en lactose est de 11 % à 56% en poids des ingrédients non aqueux de la suspension de revêtement.
